(19) Europäisches Patentamt
European Patent Office
Office européen des brevets

(11) Publication number: 0 003 221
B1

(12) # EUROPEAN PATENT SPECIFICATION

(45) Date of publication of patent specification: 21.04.82

(51) Int. Cl.³: C 07 D 311/66,
C 07 C 143/68

(21) Application number: 78101641.5

(22) Date of filing: 12.12.78

(54) **Process for the manufacture of 3,4-dihydrobenzopyran derivatives and starting materials in this process.**

(30) Priority: 30.01.78 US 873185

(43) Date of publication of application:
08.08.79 Bulletin 79/16

(45) Publication of the grant of the patent:
21.04.82 Bulletin 82/16

(84) Designated Contracting States:
CH DE FR GB NL

(56) References cited:
FR - A - 255 299

(73) Proprietor: F. HOFFMANN-LA ROCHE & CO.
Aktiengesellschaft
CH-4002 Basel (CH)

(72) Inventor: Cohen, Noal
19 Euclid Place
Montclair, N.J. (US)

(74) Representative: Cottong, Norbert A. et al,
124 Grenzacherstrasse P.O. Box 601
CH-4002 Basel (CH)

EP 0 003 221 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Process for the manufacture of 3,4-dihydrobenzopyran derivatives and starting materials in this process

The present invention is concerned with a novel process for the manufacture of compounds of the general formula

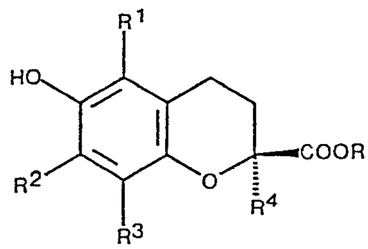

I

wherein R, $R^1$, $R^2$ and $R^3$ are hydrogen or lower alkyl and $R^4$ is lower alkyl.

U.S. Patent No. 3,947,473 discloses, inter alia, compounds of the general formula

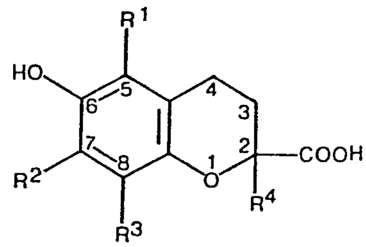

I'

wherein $R^1$, $R^2$, $R^3$ and $R^4$ have the above meanings, as well as a method for resolving these compounds into the two isomeric forms, i.e.

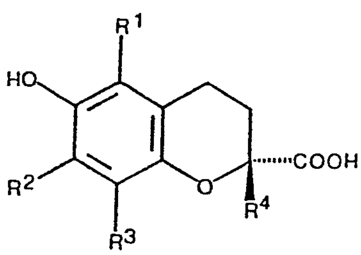

IA and

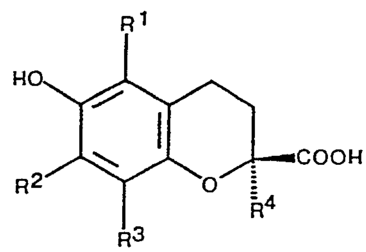

IB

wherein $R^1$, $R^2$, $R^3$ and $R^4$ have the above meanings.

In many cases, it is desired to utilize the compound of formula I' in its 2S form, i.e. the compound of formula I—B. This is especially true where $R^1$, $R^2$, $R^3$ and $R^4$ are methyl, since this compound is an intermediate for natural optically active alpha-tocopherol, i.e. alpha-tocopherol having the 2R, 4'R and 8'R configuration. Therefore, the compound of fromula I—A may be the undesired isomer and the conversion to the optically pure 2S form be desired.

In accordance with the present invention, it has now been found that the optically pure 2R compounds of formula I—A can be inverted to form the optically pure 2S-isomer. Therefore, this invention provides a method for utilizing the unwanted 2R-isomer for producing 2R, 4'R, 8'R-$\alpha$-tocopherol. In this manner, both the isomers of formula I' can be used, thereby improving the economy of the overall synthesis of 2R, 4'R, 8'R-$\alpha$-tocopherol (natural Vitamin E). Further, it provides a method for forming the antioxidants of formula I' in their optically active 2S-configuration with an improved economy.

In the structural formulae given throughout this application, the substituents attached to the molecule above the plane of the molecule are designed by ▲ and the substituents attached to the molecule below the plane of the molecule are designated by ⋮ . The numbering of the 3,4-dihydrobenzopyran ring in the compound of formula I' is given for the purpose of convenience.

As used herein, the term lower alkyl designates straight and branched chain aliphatic saturated hydrocarbon groups containing from 1 to 7 carbon atoms, such as e.g. methyl, ethyl, n-propyl, isopropyl, tert-butyl and isobutyl. The term halogen includes all four halogens, i.e. chlorine, bromine, fluorine and iodine.

The process for the manufacture of the compounds of the above formula I is characterized in that a compound of the general formula

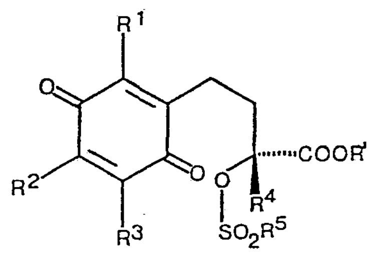

II

wherein R′ and $R^5$ are lower alkyl and $R^1$, $R^2$, $R^3$ and $R^4$ have the above meaning, is reduced in a polar organic solvent either in the presence of a base or followed by the addition of a base and that, if desired, a so obtained compound of formula I, wherein R is lower alkyl, is hydrolyzed to a compound of formula I, wherein R is hydrogen.

In accordance with the present invention, it has been found that the above mentioned reduction occurs with complete inversion of the stereo configuration. This reaction can be carried out by treating the compound of formula II with a reducing agent in the presence of a base. Any of the conventional reducing agents utilized to reduce quinones to hydroquinones can be utilized in carrying out the process of this invention. Among the preferred quinone reducing agents are included alkali metal hydrosulfites, alkali metal borohydrides and catalytic hydrogenation. Where an alkali metal borohydride is utilized, the preferred borohydrides are sodium borohydride, potassium borohydride and lithium borohydride. The preferred alkali metal hydrosulfites are sodium and potassium hydrosulfites. Among the preferred catalysts for use in catalytic hydrogenation are palladium and platinum, as well as the other metals which are conventionally used in catalytic hydrogenation. These catalysts can be used along or on conventional supports such as charcoal or carbon.

Any conventional base may be present in this reaction during the treatment of the compound of formula II with a reducing agent. On the other hand, the base can be utilized after the reduction has been carried out and also here any conventional base can be utilized. Among the preferred bases are the inorganic bases such as the alkali metal hydroxides, as well as the organic bases such as the alkali metal lower alkoxides, pyridine and lower alkylamines. Among the preferred amines are included tri-(lower alkyl) amines, such as methyldiethyl amine, triethyl amine and trimethyl amine.

This reaction is carried out in a polar solvent. Any conventional polar solvent can be utilized in carrying out this reaction. Among the conventional polar solvents are included lower alkanols or ethers such as diethyl ether and tetrahydrofuran. In carrying out this reaction, temperature and pressure are not critical and this reaction can be carried out at room temperature and atmospheric pressure. On the other hand, elevated or reduced temperatures can be utilized. Generally, it is preferred to carry out this reaction at a temperature of from about 0°C to 70°C.

The optional hydrolysis of a compound of formula I, wherein R is lower alkyl, can be carried out according to any conventional method of ester hydrolysis.

The compounds of the above formula II, which are used as starting materials in the process of the present invention, are novel compounds and form also part of the present invention.

These novel compounds of formula II can be prepared starting from compounds of formula IA via the following intermediates.

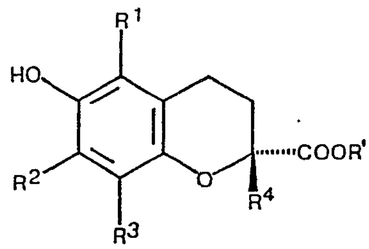

III

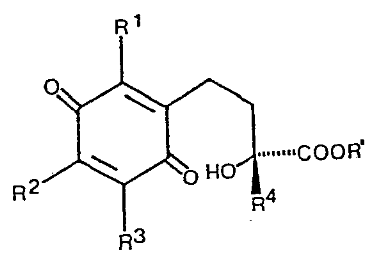

IV

wherein R′, $R^1$, $R^2$, $R^3$ and $R^4$ have the above meanings.

The conversion of a compound of formula I—A to a compound of formula III can be carried out by esterification with a lower alkanol. Any conventional method of esterifying an organic acid with a lower alkanol can be utilized to carry out this reaction. Among the preferred methods for esterification is reacting the compound formula I—A with a lower alkanol, such as methanol, in the presence of an organic acid such as p-toluene sulfonic acid. Generally, the lower alkanol can act as the solvent medium.

The conversion of a compound of formula III to a compound of formula IV can be carried out by treatment with an oxidizing agent. The oxidizing agents which can be utilized can be any conventional oxidizing agents which convert hydroquinones to benzoquinones. Among the preferred oxidizing agents are included ferric chloride, nitric acids, and ceric sulfate. Any of the conditions conventional in utilizing these oxidizing agents can be utilized in carrying out this reaction.

The compound of formula IV can be converted to the compound of formula II by treatment with a compound of the formula:

$$R^5SO_2X \qquad V$$

wherein $R^5$ has the above meaning and X is halogen.

In the compound of formula V, $R^5$ can be any lower alkyl group. The preferred compound of formula V is methane sulfonyl chloride.

Generally, the reaction of the compound of formula IV with the compound of formula V to produce a compound of the formula II is carried out in the presence of an organic amine base. Any conventional organic amine base can be utilized in carrying out this reaction. Among the preferred bases are included the tri(lower alkyl)amines and pyridine. Any conventional tri(lower alkyl)amine can be utilized in carrying out this reaction, such as methyl-diethyl amine, triethyl amine and trimethyl amine. Generally, this reaction is carried out in the presence of an inert organic solvent. Any conventional inert organic solvent can be utilized. Among the preferred inert organic solvents are the halogenated hydrocarbons such as dichloromethane and the like. In carrying out this reaction, temperatures from about −20°C to +20°C are generally utilized.

The following examples are illustrative but not limitative of the claimed invention. In these examples, the ether is diethyl ether. Furthermore, the "usual work-up" involves 3 extractions with the specified solvent. The organic extracts were then washed with water and saturated brine, dried over anhydrous magnesium sulfate, filtered and concentrated at 40—50°C under water aspirator pressure. Residues were dried to constant weight under high vacuum. Unless otherwise noted, reactions were carried out under an atmosphere of argon. Column chromatography was performed using silica gel. All temperatures are in degree centrigrade.

## Example 1

(S)-(—)-3,4-Dihydro-6-hydroxy-2,5,7,8-tetramethyl-2H-1-benzopyran-2-carboxylic acid methyl ester

A slurry of 0.358 g (1 mmole) of (R)-(—)-methyl-2-methyl-2-[(methylsulfonyl)oxy]-4-(2,4,5-trimethyl-3,6-dioxo-1,4-cyclohexadien-1-yl)-butanoate in 5 ml of methanol was stirred rapidly, at room temperature, while a solution of 0.261 g (1.5 mmoles) of sodium dithionite in 3 ml of 1N aqueous sodium hydroxide was added dropwise over a 5 minute period. The resulting mixture was stirred at room temperature for 20 minutes, then refluxed for 5 minutes. After cooling, 20 ml of water were added and the colorless slurry was filtered with suction. The solid was washed with water, then dried under high vacuum giving 0.246 g (93.2%) of (S)-(—)-3,4-dihydro-6-hydroxy-2,5,7,8-tetramethyl-2H-1-benzopyran-2-carboxylic acid methyl ester as a colorless solid; m.p. 132.5—135°; $[\alpha]_D^{25}$ −60.62° (c 3.09, $CH_3OH$).

The (R)-(—)-methyl-2-methyl-2-[(methylsulfonyl)oxy]-4-(2,4,5-trimethyl-3,6-dioxo-1,4-cyclohexadien-1-yl)-butanoate, used as starting material, was prepared as follows:

A) (R)-(+)-3,4-Dihydro-6-hydroxy-2,5,7,8-tetramethyl-2H-1-benzopyran-2-carboxylic acid methyl ester

A solution of 2 g (8 mmoles) of optically pure (R)-(+)-3,4-dihydro-6-hydroxy-2,5,7,8-tetramethyl-2H-1-benzopyran-2-carboxylic acid /$[\alpha]_D^{25}$ + 65.84° (c 1.18, $C_2H_5OH$)/ and 0.1 g of p-toluenesulfonic acid monohydrate in 40 ml of methanol was stirred and refluxed for 3.75 hours. After cooling, the solution was diluted with water and worked-up with ether in the usual manner (the ether extracts were additionally washed with saturated aqueous sodium bicarbonate solution) giving 2 g (94.7%) of (R)-(+)-3,4-dihydro-6-hydroxy-2,5,7,8-tetramethyl-2H-1-benzopyran-2-carboxylic acid methyl ester as a colorless solid; m.p. 132—134.5°; $[\alpha]_D^{25}$ + 61.4° (c 4.91, $CH_3OH$). An analytical specimen was obtained by recrystallization of a sample from aqueous methanol, as a colorless solid; m.p. 133.5—135°; $[\alpha]_D^{25}$ + 61.85 (c, 5.07, $CH_3OH$).

$B_1$) (R)-(—)-Methyl-2-hydroxy-2-methyl-4-(2,4,5-trimethyl-3,6-dioxo-1,4-cyclohexadien-1-yl) butanoate

To a stirred solution of 1.5 g (5.68 mmoles) of (R)-(+)-3,4-dihydro-6-hydroxy-2,5,7,8-tetramethyl-2H-1-benzopyran-2-carboxylic acid methyl ester in 22 ml of ether, was added a solution of 4.5 g (16.6 mmoles) of ferric chloride hexahydrate in 17 ml of water and 17 ml of methanol. The addition was carried out in approximately 6 ml portions at 0.5 hour intervals. One-half hour after the last addition, the ether layer was separated and the aqueous phase was further worked-up by ether extraction in the usual manner. There was obtained 1.5 g of a yellow oil which was chromatographed on 75 g of silica gel. Elution with 9:1 parts by volume and 4:1 parts by volume toluene/ethyl acetate yielded 1.35 g (84.9%) of (R)-(—)-methyl-2-hydroxy-2-methyl-4-(2,4,5-trimethyl-3,6-dioxo-1,4-cyclohexadien-1-yl) butanoate as a yellow oil; $[\alpha]_D^{25}$ −21.01° (c 0.96, $CHCl_3$).

$B_2$) A suspension of 2.64 g (10 mmoles) of (R)-(+)-3,4-dihydro-6-hydroxy-2,5,7,8-tetramethyl-2H-1-benzopyran-2-carboxylic acid methyl ester / $[\alpha]_D^{25}$ + 61.00° (c 3.05, $CH_3OH$) / in 25 ml of ether was vigorously stirred with ice-bath cooling while 5 ml (80 mmoles) of 70% by weight aqueous nitric acid was added dropwise over a 15 minute period. The resulting bright yellow solution was cautiously poured into excess saturated aqueous sodium bicarbonate solution. Work-up with ether in the usual manner gave 2.78 g (99.5%) of (R)-(—)-methyl 2-hydroxy-2-methyl-4-(2,4,5-trimethyl-3,6-dioxo-1,4-cyclohexadien-1-yl) butanoate as yellow oil. This material was identical with that produced as in $B_1$ above.

C) (R)-(—)-Methyl 2-methyl-2-[(methylsulfonyl)oxy]-4-(2,4,5-trimethyl-3,6-dioxo-1,4-cyclohexadien-1-yl)butanoate

To a stirred solution of 0.746 g (2.66 mmoles) (R)-(—)-methyl 2-hydroxy-2-methyl-4-(2,4,5-trimethyl-3,6-dioxo-1,4-cyclohexadien-1-yl)butanoate in 18 ml of dichloromethane, cooled in an ice-bath, were added 2.43 ml (1.77 g; 17.5 mmoles) of triethylamine followed by 1.35 ml (2.01 g; 17.5 mmoles) of methane sulphonyl chloride. The mixture was kept at 0° for 64 hours, then treated with water. The dichloromethane solution was processed in the usual manner to give 1.45 g of an oily product which was chromatographed on 75 g of silica gel. Elution with 9:1 parts by volume toluene/ethyl acetate afforded 0.69 g (72.5%) of (R)-(—)-methyl 2-methyl-2-[(methylsulfonyl)oxy]-4-(2,4,5-trimethyl-3,6-dioxo-1,4-cyclohexadien-1-yl)butanoate as a yellow solid. Recrystallization of a sample from hexane/ethyl acetate provided yellow crystals; m.p. 112—114°; $[\alpha]_D^{25}$ —5.24° (c 1.05, $CHCl_3$).

## Example 2

A mixture of 1 g (2.79 mmoles) of (R)-(—)-methyl 2-methyl-2-[(methylsulfonyl)oxy]-4-(2,4,5-trimethyl-3,6-dioxo-1,4-cyclohexadien-1-yl)butanoate (prepared according to example 1), 0.2 g of 5% palladium on charcoal and 125 ml of methanol was stirred in an atmosphere of hydrogen until gas uptake ceased. The catalyst was filtered with suction on a pad of Celite (Registered Trade Mark) and the filtrate was immediately treated with 8.3 ml (11.16 mmoles) of 1.34 M methanolic sodium methoxide. After stirring for 1 hour at room temperature, the solution was acidified with 3 N aqueous hydrochloric acid and poured into saturated brine. Work-up with ether in the usual manner gave 0.638 g of a tan solid, which was chromatographed on 25 g of silica gel. Elution with 19:1 parts by volume and 9:1 parts by volume toluene/ethyl acetate furnished 0.491 g (66.7%) of (S)-(—)-3,4-dihydro-6-hydroxy-2,5,7,8-tetramethyl-2H-1-benzopyran-2-carboxylic acid methyl ester as a colorless solid; $[\alpha]_D^{25}$ —59.93° (c 3.01, $CH_3OH$).

## Example 3

To a solution of 0.5 g (1.4 mmoles) of (R)-(—)-methyl 2-methyl-2-[(methylsulfonyl)oxy]-4-(2,4,5-trimethyl-3,6-dioxo-1,4-cyclohexadien-1-yl)butanoate (prepared according to example 1) in 30 ml of methanol, at room temperature, was added a solution of 20.2 mg (0.53 mmole) of sodium borohydride in 10 ml of methanol, dropwise, with stirring. After stirring at room temperature for 50 minutes, 5.77 ml (7.73 mmoles) of 1.34 M methanolic sodium methoxyde were added and stirring was continued for 2 hours at room temperature. The resulting solution was acidified with 1 N aqueous hydrochloric acid, then poured into saturated brine and worked-up with ether in the usual manner. The crude, crystalline product (0.34 g) was chromatographed on 20 g of silica gel. Elution with 19:1 parts by volume toluene/ethyl acetate gave 0.311 g (84.1%) of (S)-(—)-3,4-dihydro-6-hydroxy-2,5,7,8-tetramethyl-2H-1-benzopyran-2-carboxylic acid methyl ester as a colorless solid; m.p. 131—134°; $[\alpha]_D^{25}$ —60.46° (c 3.88, $CH_3OH$).

**Claims**

1. Process for the manufacture of 3,4-dihydrobenzopyran derivatives of the general formula

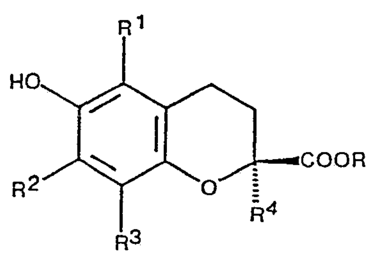

I

wherein R, $R^1$, $R^2$ and $R^3$ are hydrogen or lower alkyl and $R^4$ is lower alkyl, characterized in that a compound of the general formula

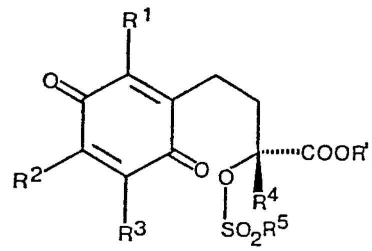

II

wherein R' and $R^5$ are lower alkyl and $R^1$, $R^2$, $R^3$ and $R^4$ have the above meaning, is reduced in a polar organic solvent either in the presence of a base or followed by the addition of a base and that, if desired, a so obtained compound of formula I, wherein R is lower alkyl, is hydrolyzed to a compound of formula I, wherein R is hydrogen.

2. Process according to claim 1, characterized in that there is used as a base an alkali metal hydroxide, an alkali metal alkoxide, a tri(lower alkyl)amine or pyridine.

3. Process according to claim 1 or 2, characterized in that the reduction is carried out by catalytic hydrogenation.

4. Process according to claim 1 or 2, characterized in that the reduction is carried out with an alkali metal borohydride or an alkali metal hydrosulfite.

5. Process according to any one of claims 1 to 4, characterized in that there is used a starting material of formula II, wherein $R^1$, $R^2$, $R^3$ and $R^4$ are methyl.

6. Process according to any one of claims 1 to 5, characterized in that there is used a starting material of formula II, wherein $R^5$ is methyl.

7. A compound of the general formula

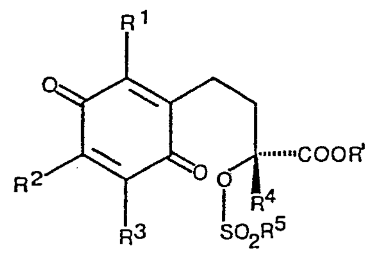

II

wherein $R^1$, $R^2$ and $R^3$ are hydrogen or lower alkyl and R', $R^4$ and $R^5$ are lower alkyl.

8. A compound according to claim 7, wherein $R^1$, $R^2$, $R^3$ and $R^4$ are methyl.

9. (R)-(—)-methyl 2-methyl-2-[(methylsulfonyl)oxy]-4-(2,4,5-trimethyl-3,6-dioxo-1,4-cyclohexadien-1-yl)butanoate.

## Revendications

1. Procédé de préparation de dérivés de 3,4-dihydrobenzopyrane de formule générale:

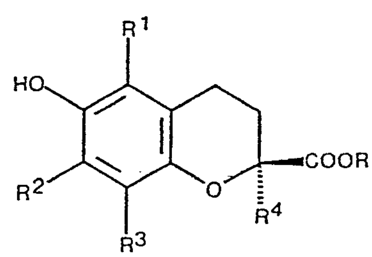

I

où R, $R^1$, $R^2$ et $R^3$ sont des atomes d'hydrogène ou des groupes alcoyle inférieurs et où $R^4$ est un groupe alcoyle inférieur, caractérisé en ce qu'on réduit le composé de formule générale:

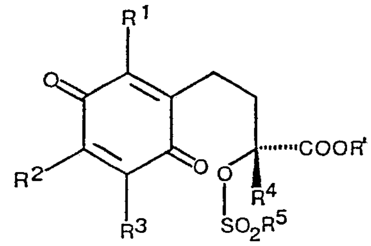

II

où R' et $R^5$ sont des groupes alcoyle inférieurs et où $R^1$, $R^2$, $R^3$ et $R^4$ ont la signification donnée ci-dessus, dans un solvant organique polaire, en présence d'une base ou en ajoutant ensuite une base, et en ce que, si on le désire, on hydrolyse un composé de formule I ainsi obtenu, où R est un groupe alcoyle inférieur, pour donner un composé de formule I, où R est un atome d'hydrogène.

2. Procédé selon la revendication 1, caractérisé en ce qu'on utilise comme base un hydroxyde de métal alcalin, un alcoolate de métal alcalin, une tri(alcoyle inférieur)amine ou la pyridine.

3. Procédé selon la revendication 1 ou 2, caractérisé en ce que la réduction s'effectue par hydrogénation catalytique.

4. Procédé selon la revendication 1 ou 2, caractérisé en ce que la réduction s'effectue avec un borohydrure de métal alcalin ou un hydrosulfite de métal alcalin.

5. Procédé selon l'une quelconque des revendications 1 à 4, caractérisé en ce qu'on utilise un produit de départ de formule II, où $R^1$, $R^2$, $R^3$ et $R^4$ représentent un groupe méthyle.

6. Procédé selon l'une quelconque des revendications 1 à 5, caractérisé en ce qu'on utilise un produit de départ de formule II où $R^5$ est un groupe méthyle.

7. Composé de formule générale:

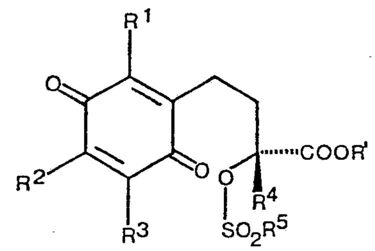

II

où $R^1$, $R^2$ et $R^3$ sont des atomes d'hydrogène ou des groupes alcoyle inférieurs et où R', $R^4$ et $R^5$ sont des groupes alcoyle inférieurs.

8. Composé selon la revendication 7, où $R^1$, $R^2$, $R^3$ et $R^4$ représentent un groupe méthyle.

9. Le (R)-(—)-méthyl-2-méthyl-2-[(méthylsulfonyl)oxy]-4-(2,4,5-triméthyl-3,6-dioxo-1,4-cyclohexadièn-1-yl)butanoate.

## Patentansprüche

1. Verfahren zur Herstellung von 3,4-Dihydrobenzopyran-derivaten der allgemeinen Formel

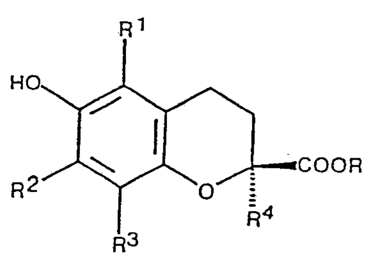

I

worin R, $R^1$, $R^2$ und $R^3$ Wasserstoff oder niederes Alkyl und $R^4$ niederes Alkyl bedeuten, dadurch gekennzeichnet, dass man eine Verbindung der allgemeinen Formel

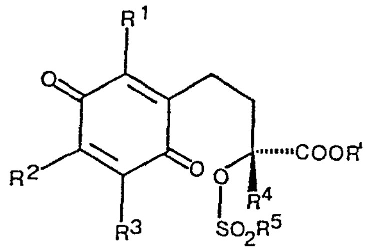

II

worin R' und $R^5$ niederes Alkyl bedeuten und $R^1$, $R^2$, $R^3$ und $R^4$ die obige Bedeutung haben, in einem polaren, organischen Lösungsmittel, entweder in Gegenwart einer Base oder gefolgt von der Zugabe einer Base, reduziert und dass man, gewünschtenfalls, eine so erhaltene Verbindung der Formel I, worin R niederes Alkyl bedeutet, zu einer Verbindung der Formel I, worin R Wasserstoff bedeutet, hydrolysiert.

2. Verfahren gemäss Anspruch 1, dadurch gekennzeichnet, dass man als Base ein Alkalimetallhydroxid, ein Alkalimetallalkoxid, ein Tri-niederes Alkylamin oder Pyridin verwendet.

3. Verfahren gemäss Anspruch 1 oder 2, dadurch gekennzeichnet, dass man die Reduktion durch katalytische Hydrierung durchführt.

4. Verfahren gemäss Anspruch 1 oder 2, dadurch gekennzeichnet, dass man die Reduktion mit einem Alkalimetallborhydrid oder einem Alkalimetallhydrogensulfit durchführt.

5. Verfahren gemäss einem der Ansprüche 1 bis 4, dadurch gekennzeichnet, dass man ein Ausgangsmaterial der Formel II verwendet, worin $R^1$, $R^2$, $R^3$ und $R^4$ Methyl bedeuten.

6. Verfahren gemäss einem der Ansprüche 1 bis 5, dadurch gekennzeichnet, dass man ein Ausgangsmaterial der Formel II verwendet, worin $R^5$ Methyl bedeutet.

7. Eine Verbindung der allgemeinen Formel

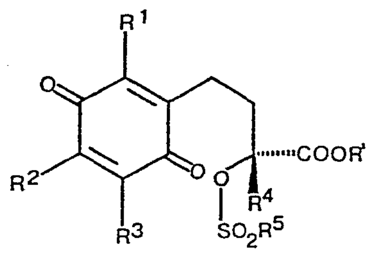

II

worin $R^1$, $R^2$ und $R^3$ Wasserstoff oder niederes Alkyl und R', $R^4$ und $R^5$ niederes Alkyl bedeuten.

8. Eine Verbindung gemäss Anspruch 7, worin $R^1$, $R^2$, $R^3$ und $R^4$ Methyl bedeuten.

9. (R)-(—)-Methyl-2-methyl-2-[(methylsulfonyl)oxy]-4-(2,4,5-trimethyl-3,6-dioxo-1,4-cyclohexadien-1-yl)butanoat.